# EUROPEAN PATENT APPLICATION

(11) **EP 4 830 654 A2**
(43) Date of publication of application: **28.10.2026**
(21) Application number: 26204598.2
(22) Date of filing: 24.03.2022
(51) Int. Cl.: A61B 18/14

(54) **CONTROLLING CONDUCTIVE PATHS BETWEEN ELECTRODES IN ELECTROSURGICAL INSTRUMENTS AND RELATED SYSTEMS AND METHODS**

(30) Priority: 25.03.2021 US 202163165940 P
(62) Document number(s) of the earlier application(s) in accordance with Art. 76 EPC: 22716658.4 / 4 312 847
(71) Applicant: Intuitive Surgical Operations, Inc., Sunnyvale, CA 94086 (US)
(72) Inventor: SHAH, Jignesh M., Sunnyvale, 94086 (US); LEE, Chien-Hsun, Sunnyvale, 94086 (US); MARION, Duane W., Sunnyvale, 94086 (US); SCHOETTGEN, Benjamin J., Sunnyvale, 94086 (US)
(74) Representative: Mathys & Squire

(57) **Abstract**

An electrosurgical instrument may comprise electrodes provided on the working surfaces of each jaw member that can be set to different electrical potentials and/or polarities to perform electrosurgical operations, such as sealing and cutting. Different combinations of electrodes can be selectively activated to create different pathways for the transfer of electrical energy through the material, to achieve the desired effects

## Description

### TECHNICAL FIELD

Aspects of the present disclosure relate to electrosurgical instruments and related systems and methods. More specifically, aspects of the present disclosure relate to electrode configurations and operating states thereof for electrosurgical instruments.

### INTRODUCTION

Remotely controlled surgical instruments, including both manual, laparoscopic instruments and computer-assisted, teleoperated surgical instruments (sometimes referred to as robotic surgical instruments), are often used in minimally invasive medical procedures. For example, in teleoperated surgical systems, surgeons manipulate input devices at a surgeon console, and those "master" inputs are passed to a patient side cart that interfaces with one or more remotely controlled surgical instruments coupled to the patient side cart. Based on the surgeon inputs at the surgeon console, the one or more remotely controlled surgical instruments are actuated at the patient side cart to operate on the patient, thereby creating a master-slave control relationship between the surgeon console and the surgical instrument(s) at the patient side cart.

Minimally-invasive surgical instruments can take the form of electrosurgical instruments configured to deliver electrical energy to material such as tissue, or a tissue-like material for testing purposes. Electrosurgical instruments are coupled to an electrosurgical energy generating unit (ESU) that generates and supplies electrical current to the electrosurgical instrument so that an electrosurgical energy can be applied to tissue using an end effector of the electrosurgical instrument. An end effector can include one or more electrodes configured to supply energy at the desired site. Some electrosurgical instrument end effectors include gripping end effectors comprising a pair of opposing jaws fitted with electrodes that can perform cutting and/or sealing operations on, for instance, vessels and other types of tissue. Cutting and sealing operations generally rely on electrical energy from an ESU that is converted to thermal energy when the gripping end effector grip a section of tissue. Further, such electrosurgical instruments utilize a bipolar mode of energy delivery in that an electrode on one of the opposing jaws serves as an active, energy delivery electrode and an electrode on the other of the opposing jaws serves as a dispersive (return) electrode to safely return the delivered energy to the ESU or another ground.

When using the same bipolar electrosurgical instrument to both seal and cut tissue, the energy profiles that are needed may differ to achieve the desired effects. In some cases, cutting tissue using electrosurgical energy can pose issues in achieving a clean cut (e.g., without jaggedness in the tissue at the cut line). Thus, it is desirable to provide electrode arrangements and control over the electrical pathways between the electrodes that provide for robust cutting and sealing procedures to be performed using the same electrosurgical instrument.

Thus, there exists a continued need to improve upon electrosurgical instruments and related systems and methods for delivering electrosurgical energy to perform various surgical procedures and, in particular, for an electrosurgical instrument to accurately and reliably perform both sealing and cutting procedures.

### SUMMARY

Exemplary embodiments of the present disclosure may solve one or more of the above-mentioned technical challenges and/or may demonstrate one or more of the above-mentioned desirable features. Other features and/or advantages may become apparent from the description that follows.

In accordance with at least one exemplary embodiment, the present disclosure contemplates an electrosurgical system comprising an electrosurgical energy supply unit, and an electrosurgical instrument electrically coupled to the electrosurgical energy supply unit. The electrosurgical instrument comprises a first jaw member comprising a first electrode and a second electrode, and a second jaw member comprising a third electrode. The electrosurgical energy supply unit is configured to control operation of the first, second, and third electrodes such that in a first operational state, a first electric potential difference is established between the first electrode and the third electrode, the second electrode is at a neutral potential, and electrical energy is supplied to the first electrode and returned from the third electrode, and in a second operational state, a second electric potential difference is established between the second electrode and the first electrode, the third electrode is at the neutral potential, and electrical energy is supplied to the second electrode and returned from the first electrode.

In accordance with at least one exemplary embodiment, the present disclosure contemplates a method of controlling an electrosurgical instrument, the method comprising selectively operating the electrosurgical instrument between first and second operational states, the electrosurgical instrument comprising a pair of opposing jaws. Operating the electrosurgical instrument in the first operational state comprises routing electrical energy between a first pair of electrodes and through tissue grasped between the jaws, each electrode of the first pair located on differing jaws of the pair of opposing jaws. Operating the electrosurgical instrument in the second operational state comprises routing electrical energy between a second pair of electrodes and through the tissue grasped between the jaws, each electrode of the second pair located on a same jaw of the pair of opposing jaws.

In accordance with at least one exemplary embodiment, the present disclosure contemplates a method of controlling an electrosurgical instrument, the method comprising imparting a first cutting signal between a first set of electrodes of an end effector of an electrosurgical instrument, wherein the first set of electrodes are provided on a single jaw member of the end effector, imparting a second cutting signal between a second set of electrodes of the end effector, wherein the second set of electrodes are provided on at least two different jaw members of the end effector, and wherein the first and second set of electrodes have at least one electrode in common, and alternating between imparting the first and second cutting signals between the first and second sets of electrodes respectively.

Additional objects, features, and/or advantages will be set forth in part in the description which follows, and in part will be obvious from the description, or may be learned by practice of the present disclosure and/or claims. At least some of these objects and advantages may be realized and attained by the elements and combinations particularly pointed out in the appended claims.

It is to be understood that both the foregoing general description and the following detailed description are exemplary and explanatory only and are not restrictive of the claims; rather the claims should be entitled to their full breadth of scope, including equivalents.

### BRIEF DESCRIPTION OF THE DRAWINGS

The present disclosure can be understood from the following detailed description, either alone or together with the accompanying drawings. The drawings are included to provide a further understanding of the present disclosure, and are incorporated in and constitute a part of this specification. The drawings illustrate one or more exemplary embodiments of the present teachings and together with the description serve to explain certain principles and operation.
FIG. 1 is an illustrative perspective view of a surgical instrument.
FIG. 2 is an illustrative plan view of a minimally invasive teleoperated surgical system.
FIG. 3 is a block diagram representing an electrosurgical energy generation unit (ESU) in accordance with some embodiments.
FIG. 4 is an illustrative perspective view of a pair of jaws of an electrosurgical instrument end effector in an open position and comprising an arrangement of electrodes and corresponding circuitry configured to achieve provide a tissue sealing and tissue cutting operational state of the electrosurgical instrument in accordance with some embodiments.
FIG. 5 is a distal end view of the pair of jaws of FIG. 4 shown in a closed position with biological tissue grasped between them in accordance with some embodiments.
FIGS. 6A-6D are distal end views of the pair of end effector jaws in different operating states in accordance with some embodiments.
FIG. 7 is a method for controlling electrosurgical instruments in accordance with some embodiments.

### DETAILED DESCRIPTION

This description and the accompanying drawings that illustrate exemplary embodiments should not be taken as limiting. Various mechanical, compositional, structural, electrical, and operational changes may be made without departing from the scope of this description and the claims, including equivalents. In some instances, well-known structures and techniques have not been shown or described in detail so as not to obscure the disclosure. Like numbers in two or more figures represent the same or similar elements. Furthermore, elements and their associated features that are described in detail with reference to one embodiment may, whenever practical, be included in other embodiments in which they are not specifically shown or described. For example, if an element is described in detail with reference to one embodiment and is not described with reference to a second embodiment, the element may nevertheless be claimed as included in the second embodiment.

For the purposes of this specification and appended claims, unless otherwise indicated, all numbers expressing quantities, percentages, or proportions, and other numerical values used in the specification and claims, are to be understood as being modified in all instances by the term "about," to the extent they are not already so modified. Accordingly, unless indicated to the contrary, the numerical parameters set forth in the following specification and attached claims are approximations that may vary depending upon the desired properties sought to be obtained. At the very least, and not as an attempt to limit the application of the doctrine of equivalents to the scope of the claims, each numerical parameter should at least be construed in light of the number of reported significant digits and by applying ordinary rounding techniques.

It is noted that, as used in this specification and the appended claims, the singular forms "a," "an," and "the," and any singular use of any word, include plural referents unless expressly and unequivocally limited to one referent. As used herein, the term "include" and its grammatical variants are intended to be non-limiting, such that recitation of items in a list is not to the exclusion of other like items that can be substituted or added to the listed items.

In accordance with various exemplary embodiments, the present disclosure contemplates bipolar electrosurgical instruments comprising gripping end effectors that are configured to deliver electrical energy to perform electrosurgical procedures such as sealing (cauterizing) and cutting (incision) of tissue, including but not limited to blood vessels for example. For training and/or testing purposes, the exemplary surgical instruments described herein can also be used on materials having properties similar to tissue. In various exemplary embodiments described herein, a gripping end effector of a surgical instrument can include opposing jaws, with the jaws being provided with an arrangement of electrodes on their respective working surfaces that can be operated to provide different sets of active and return electrodes, thereby providing the ability to use the electrosurgical instrument to achieve both sealing and cutting functionality. In accordance with some embodiments, the circuitry to the electrodes may be such that various electrodes of the arrangement can be set to a positive, negative, or neutral potential in such a manner so as to enable at least one electrode to serve as an active electrode and another as a return electrode. Tissue gripped between the jaws can complete the circuit between the active and return electrodes, thereby causing electrical energy to flow along conductive pathways, into the tissue. Depending on the intended procedure to be performed, different electric potential differences can be established between one or more active electrodes and one or more return electrodes, such as, for example, during a sealing procedure versus a cutting procedure. In some embodiments, the conductive pathways can be selectively modified by changing the selection of active and return electrode pairs, including for example, during the procedure, allowing for robust operation of the electrosurgical instrument to achieve both accurate cutting and sealing. In some embodiments, while in a cutting operational mode, the electrical pathways can be altered, by dynamically changing which electrode(s) of the arrangement are being used as a return electrode for an active cutting electrode. Altering the electrical pathways through the tissue during a cutting operation can promote the ability to achieve a clean and accurate cut through tissue.

In various exemplary embodiments described herein, the jaws can carry the electrodes at different sections of the jaws. For example, a working surface of a first jaw of a pair of opposing jaws can have at least a first and second electrodes and a working surface of a second jaw of the pair of opposing jaws can have at least a third electrode. In this way, an electric potential difference can be generated between the first and third electrodes to perform a sealing procedure and an electric potential difference can be generated between the second and third electrodes to perform a cutting procedure. In this way, the first electrode can be configured to function as an active sealing electrode and the second electrode to function as an active cutting electrode, while the third electrode can be configured to function as a return electrode in either the sealing or the cutting procedure. The third electrode can be provided at a location of the working surface of the second jaw that is generally aligned under the first electrode to permit a sealing procedure to be performed while the jaws grasp tissue. A variety of arrangements and numbers of electrodes can be implemented and are considered within the scope of the disclosure, as would be understood by those skilled in the art based on the disclosure and principles of operation described herein. In addition or in lieu of utilizing the third electrode as the return electrode during a cutting operation, the present disclosure contemplates that during a cutting operation, the third electrode is set to neutral and an electric potential difference is generated between the second and first electrodes of the first jaw to perform a cutting procedure. In this way, the second electrode functions as the active cutting electrode and the first electrode functions as the return electrode for the cutting procedure, whereas it is utilized as the active sealing electrode during a sealing procedure.

In various operating states, an electric potential difference between the first electrode and the third electrode sufficient to achieve a sealing flow (i.e., a flow that induces a sealing effect) of electrical energy through the tissue can be established, and an electric potential difference sufficient to achieve a cutting flow (i.e., a flow that induces a cutting effect) of electrical energy can be selectively established between the second electrode and the first electrode of the first jaw or between the second electrode and the third electrode of the second jaw. Establishing the cutting electric potential difference between the second and first electrodes (i.e., on the same (first) jaw) may require less energy due to the relative proximity of the first and second electrodes than establishing the cutting electric potential difference between the second and third electrodes (i.e., on opposite (first and second) jaws), thereby reducing a total amount of energy required for a cutting operation. In some embodiments, it is contemplated to alternate between establishing an electric potential difference between the second and first electrodes and between the second and third electrodes during a cutting procedure. It is further contemplated that the alternating can occur multiple times during the cutting procedure, the frequency of which can be adjusted. In some embodiments, the adjustment of the frequency can be dynamic and based on one or more sensed parameters, such as, for example, measured impedance of tissue grasped between the jaws and intended to be cut, while in other embodiments the frequency can be preset or altered at preset patterns or times during a cutting procedure. By alternating the pair of electrodes (one functioning as active and one as return) between which the cutting electric potential difference is established, the cutting procedure can occur more accurately and efficiently. The ability to selectively modify the pair of electrodes that are used to establish the cutting electric potential difference and/or the frequency of alternating between different pairs of electrodes, and further to do so based on sensed parameters, can further reduce the total amount of energy (and thus improve efficiency) for cutting operations, reduce undesirable arcing of electrosurgical energy, and/or promote accurate (e.g., clean and complete) cuts of tissue that do not leave tissue fragments or uncut pieces.

For ease of description, various exemplary embodiments set forth below describe electrosurgical instruments that are remotely controlled (e.g., via teleoperation or manually) by a surgeon, and powered by energy supply sources or generators (e.g., ESUs). In various exemplary embodiments, the energy supply source may supply electrosurgical energy to the circuit comprising the electrodes at voltages ranging from 10 volts (V) to 1000V, and currents ranging from 0.1 amps (A) to 10A. For example, a sealing voltage can range from 10V to 200V, a sealing current can range from 0.2A to 4A, a cutting voltage can range from 50Vto 1000V, and a cutting current can range from 0.1A to 2A. Further in various exemplary embodiments, power from the energy generation source may range from 10 Watts (W) to a few hundred Watts, for example from 10 Watts to 300 Watts, or from 10 Watts, to 250 Watts, or from 10 Watts to 200 Watts. For example, a typical range for a sealing power can be from 25W to 60W, and a typical range for a cutting power can be from 25W to 100W. Further, depending on whether a sealing or cutting procedure is to occur, and for the latter which pair(s) of electrodes are functioning as the active/return electrodes for the cutting procedure, the present disclosure contemplates a control system that will modify the voltage, current, and/or power of an ESU to provide desirable settings.

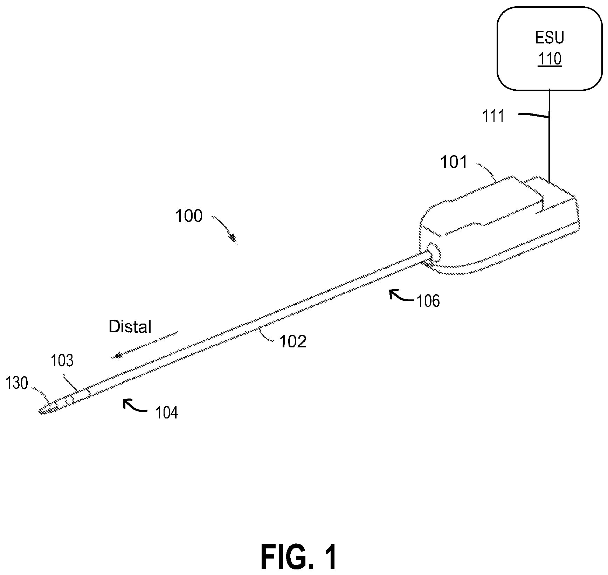

FIG. 1 is a perspective, diagrammatic view of an electrosurgical instrument 100 operably coupled to an electrical energy generation source 110 (electrical energy source 110). The electrosurgical instrument 100 includes an elongated hollow tubular shaft 102 having a centerline longitudinal axis, a distal (first) end portion 104 for insertion into a patient's body cavity, and a proximal (second) end portion 106 to which a force transmission mechanism 101 is coupled. The electrosurgical instrument 100 may be used to carry out a variety of minimally-invasive surgical, including diagnostic, procedures. The electrosurgical instrument end effector 130 can include one or more functional mechanical degrees of freedom. In the embodiment of FIG. 1, the end effector 130 comprises jaws that open and close. To open and close, both jaws may be movable or one jaw may be stationary and the other moveable. In some embodiments, the electrosurgical instrument 100 can include one or more optional wrist members 103 coupling the end effector 130 to the shaft and configured to articulate relative to the shaft in pitch and/or yaw motion. Transmission mechanism 101 can include mechanisms to impart motion to the end effector 130, such as opening or closing of jaws, and to the optional wrist member(s) 103. Mechanisms can include any combination of actuator motors, actuation elements, force transmission cables, etc.

Transmission mechanism 101 can further be configured to receive inputs (e.g., via various drives, levers, buttons etc.) depending on whether the instrument is configured for manual operation or computer-assisted, teleoperation). The inputs in tum drive mechanisms in the housing of the transmission mechanism 101 that are operably coupled to the actuation elements to transmits force from the transmission mechanism 101 along the shaft 102 to control movement of the end effector and/or wrist member(s). Transmission mechanism 101 may include a connection interface (not shown) configured to connect to a cable 111 to operably couple the instrument to an electrical energy source 110. The instrument 100 can further include one or more conduits (e.g., electrical cables) to transmit electrical signals to one or more electrodes of end effector 130 to perform electrosurgical procedures on tissue grasped between jaws of end effector 130, as will be explained further below. Such conduits can operably couple to the electrical interface at the transmission mechanism 101, that in turn is electrically coupled to the electrical energy source 110 and routed through the shaft 102 to the electrodes at the end effector 130. In various exemplary embodiments, the electrical energy source 110 can be an electrosurgical supply unit (ESU), which can provide a variety of controls and types of energy to the electrosurgical instrument 100, and with which those having ordinary skill in the art are generally familiar. As will become apparent from the description that follows, an ESU in accordance with embodiments of the present disclosure may be programmed with settings to alternate a current supply to electrodes in accordance with aspects of the present disclosure to achieve desired cutting procedures.

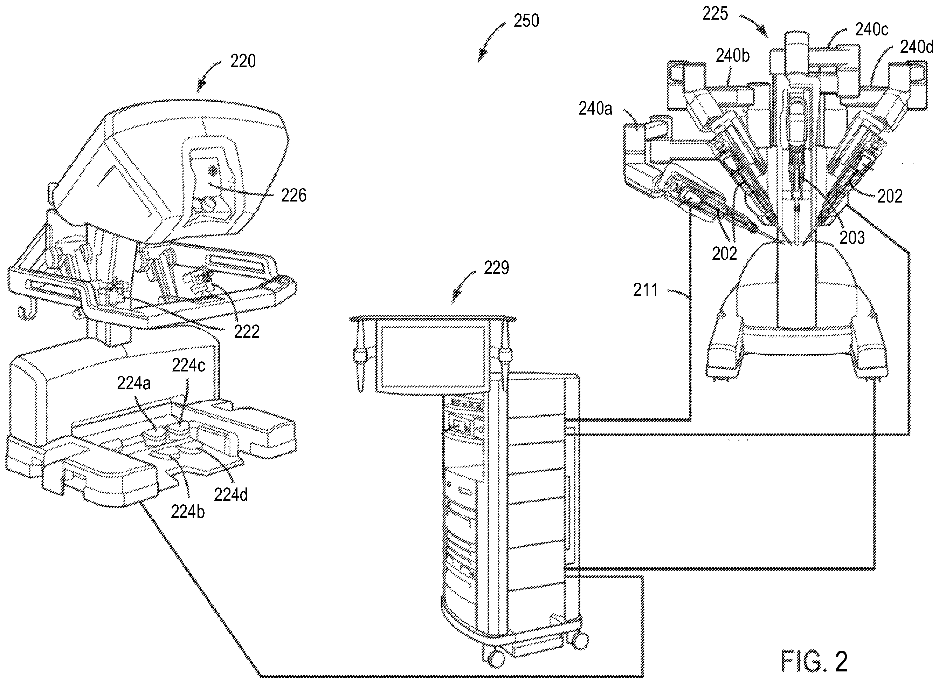

As mentioned above, electrosurgical instruments according to embodiments of the disclosure, such as the electrosurgical instrument of FIG. 1, can be manually operated, such that various inputs are provided manually at the transmission mechanism or can be configured for a teleoperated surgical system such that the instrument is operably coupled to a patient side manipulator system driven in response to master controls at a surgeon console. FIG. 2 is an illustrative plan view of a minimally invasive teleoperated surgical system 250 for performing a minimally invasive diagnostic or surgical procedure. The system includes a surgeon's console 220 for use by a surgeon during the procedure. The minimally invasive teleoperated surgical system 250 further includes a patient-side cart 225 (also referred to as a manipulator system) and an electronics cart 229 (also referred to as an auxiliary function cart). The patient-side cart 225 can manipulate at least one surgical instrument through a minimally invasive incision in the body of a patient while the surgeon views the surgical site through the surgeon's console 220. One or more surgical instruments 202, which can include an electrosurgical instrument such as electrosurgical instrument 100, can be mounted to the patient-side cart 225 to perform a minimally-invasive procedure.

An image of the surgical site can be obtained by an endoscope 203, such as a stereoscopic endoscope, which also may be mounted at and manipulated by the patient-side cart 225 to orient the endoscope 203. Computer processors located on the electronics cart 229 may be used to process the images of the surgical site for subsequent display to the surgeon through the surgeon's console 220. The surgeon's console 220 and the electronics cart 229 may be coupled to one another and/or to the patient-side cart 225 and/or directly to instruments 202 through various connections 211 (e.g., electrical cables, wires, or other connections) illustrated schematically as lines in FIG. 2. In some embodiments, stereoscopic images may be captured, which allow the perception of depth during a surgical procedure. The surgeon's console 220 can include a viewer display 226 for presenting the surgeon with a coordinated stereoscopic view of the surgical site that enables depth perception. The surgeon's console 220 further includes one or more hand-operated control inputs 222 to receive the larger-scale hand control movements and includes one or more foot pedal controls 224a-224d. One or more surgical instruments 202 installed for use on the patient-side cart 225 move in smaller-scale distances in response to larger-scale manipulation of the one or more control inputs 222. The control inputs 222 may provide the same mechanical degrees of freedom as their associated surgical instruments 202 to provide the surgeon with telepresence, or the perception that the control inputs 222 are integral with the instruments 202 so that the surgeon has a strong sense of directly controlling the instruments 202. To this end, position, force, and tactile feedback sensors (not shown) may be employed to transmit position, force, and tactile sensations from the surgical instruments 202 back to the surgeon's hands through the control inputs 222, subject to communication delay constraints.

The patient-side cart 225 includes mechanical manipulation arms 240a-240d (collectively, manipulation arms 240). A surgical instrument manipulator, which includes motors to control instrument motion, is mounted at the end of each manipulation arm 240. Additionally, each manipulation arm 240 can optionally include one or more setup joints (e.g., unpowered and/or lockable) that are used to position the attached surgical instrument manipulator in relation to the patient for surgery. The various components of the teleoperated surgical system can vary, as those having ordinary skill in the art would recognize, with FIG. 2 being nonlimiting and exemplary.

Other nonlimiting, exemplary embodiments of teleoperated surgical systems with which the electrosurgical instruments and principles of operation disclosed herein may be utilized include those such as the da Vinci^{®} Surgical Systems commercialized by Intuitive Surgical, Inc., of Sunnyvale, California However, persons having ordinary skill in the art will appreciate that the present disclosure can be applied to a variety of surgical systems including automated or manual (hand-held) laparoscopic surgical systems.

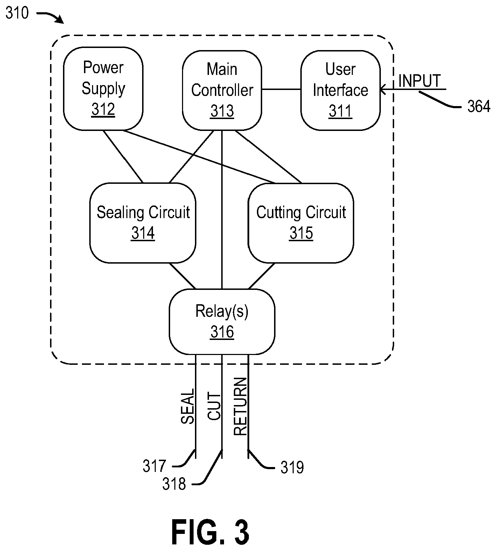

FIG. 3 is a block diagram representing an electrosurgical energy generation unit (ESU) 310 in accordance with some embodiments. The ESU 310 can be used as the electrical energy generation source in the embodiment of FIG. 1. In an exemplary embodiment, ESU 310 can be configured to generate different signals for enabling an operably coupled electrosurgical instrument to perform different operations, such as sealing and cutting. The signals can include alternating current (A/C) or direct current (D/C) signals, and any combination of A/C and D/C signals. Further, delivering sealing, cutting, or any other signal to end-effectors of a surgical instrument can include applying different voltages to electrodes provided within the end-effectors so as to establish electric potential differences. For example, signals 317, 318, and 319 can be delivered (and returned) to relay(s) 316 or any other component of ESU 310 via electrical transmission conduits, such as wires, etc. In an exemplary embodiment, a high frequency (HF) A/C sealing signal 317 can be delivered to a one or more pairs of sealing electrodes provided on opposing jaw members of an electrosurgical instrument. Further, a HF A/C cutting signal 318 can be delivered to one or more pairs of cutting electrodes on the same and/or different jaw members. Each of the one or more pairs of electrodes are configured to establish the electric potential difference between them based on the voltage settings controlled by the ESU, with one of the electrodes of the pair being supplied with energy and functioning as the active electrode and the other functioning as the return electrode for returning a portion of electrical energy as a return signal 319.

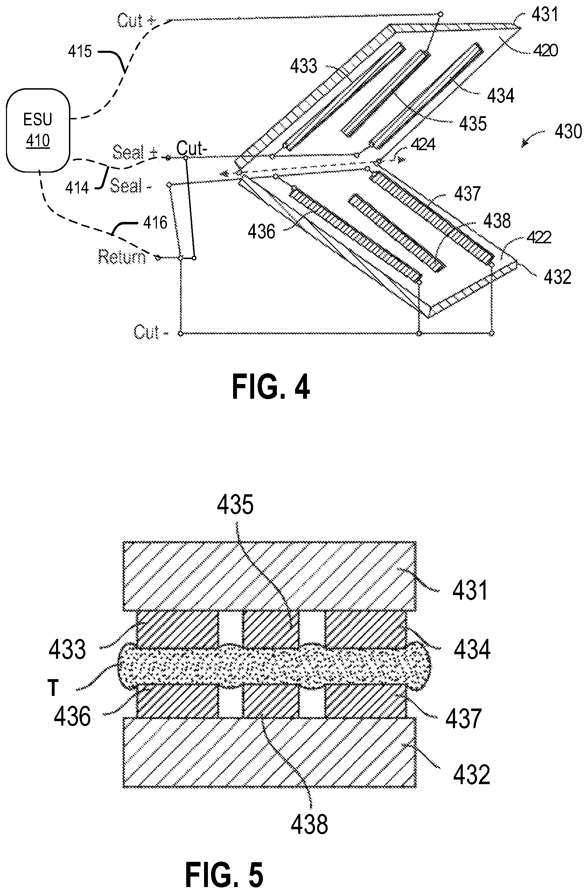

In some embodiments, the one or more pairs of sealing electrodes and one or more pairs of cutting electrodes can share at least one electrode in common, which can be operated as the return electrode. In other embodiments, an electrode can function as an active electrode or as a return electrode depending on whether a sealing procedure or a cutting procedure is occurring. In other words, in one operational state of the electrosurgical instrument, an electrode may be the active electrode of a pair of active/return electrodes and in another operational state of the electrosurgical instrument, the electrode may be a return electrode of a pair of active/return electrodes. Additional details regarding electrode arrangements and operational states of an electrosurgical instrument according to some embodiments of the present disclosure are described further below with reference to the embodiments of FIGS. 5A-5D.

Power supply 312 can include one or more power supplies configured to supply any combination of A/C and D/C voltages to sealing circuit 314 and cutting circuit 315. Power supply 312, sealing circuit 314, and cutting circuit 315 can include one or more AC-to-DC or DC-to-AC power supplies, buck regulator circuits, output transformers, and terminals that are configured to provide signals to respectively establish cutting electric potential difference or sealing electric potential difference to different pairs of electrodes of an end-effector. For example, one or more micro-controllers within sealing circuit 314 and/or cutting circuit 315 are configured to provide pulse width modulated (PWM) signals and to produce a control signal to control switching, to determine HF signal waveform patterns, including duty cycle and frequency, to determine impedances between different pairs of electrodes based upon the monitored voltage and current across them, and adjust duration and frequency of switching between operating states, as further described herein.

Main controller 313 is configured to receive inputs 364 via user interface 311, and direct operations of the sealing circuit 314 and the cutting circuit 315. In exemplary embodiments, main controller 313 can make decisions regarding switching cutting circuit 315 on and off to generate cut pulses. Further, main controller 313 can control the relay(s) 316 to direct seal, cut, and/or return signals 317-319 to and from different combinations of electrodes in top and/or bottom jaws. The main controller 313 can also receive information from sensors in the sealing circuit 314 and cutting circuit 315 to determine impedance and phase, which can be used to determine how to control operation of relay(s) 316 such as activating, stopping, or closing relay(s) 316.

Further, user interface 311 may be operably coupled to input devices, such as foot pedals or buttons or the like, to receive user input 364 to start and stop sealing and cutting activities and to indicate parameters to use for sealing and cutting signal waveforms such as voltage, current, signal frequency, and dwell time, for example. Those having ordinary skill in the art are familiar with such input devices that provide commands to control the ESU 310 to supply the desired electrosurgical functionality to the electrosurgical instrument. In embodiments using telesurgical systems, such input devices may be provided, for example, at a surgeon console. In other embodiments, such input devices may be provided through the transmission mechanism at the proximal end portion of the instrument or through other stand-alone mechanisms operably coupled to the electrosurgical instrument and to the ESU 310.

The user interface 311 can also provide feedback information, for example, at a display on the surgeon console, an auxiliary function cart, and/or an ESU, to the user such as amount of power delivered, whether a seal was successfully completed, whether an error condition occurred. A surgeon may use the user interface to provide user input to select voltage and current levels or sealing signal patterns and cutting signal patterns based upon requirements of a particular patient or surgical procedure, for example. Although the sealing and cutting signals are periodic signals that are in phase with each other, they typically have different peak-to-peak voltage potentials. In general, impedance is lower during a sealing activity than during a cutting activity due to the higher impedance associated with the plasma discharge required to resect tissue. Thus, in general, a lower voltage ordinarily may be used during sealing than is used during a cutting. In some embodiments, for example, the peak-to-peak voltage for a sealing activity is in a range of approximately 75V-150V and the peak-to-peak voltage for a cutting activity is in a range of approximately 400-800V. Conversely, in general, a higher current may be used during sealing than is used during a cutting.

In some exemplary embodiments, sealing circuit 314 is pre-configured or programmed (via, for example, user interface 311 or main controller 313) for initial electrode polarities, supplied waveforms and/or voltages to perform a sealing procedure. Then, when the sealing procedure is completed, the electrode polarities settings, etc. are modified to perform a cutting procedure. Alternatively or in addition to such user control, sensors may be used to sense differing stages of the seal and cut procedure. For example, a sensor may be used to determine when the sealing procedure is completed, and to promulgate an automatic initiation of the cutting procedure thereafter. The sensors may measure, for example, a tissue impedance and/or a phase angle of electrical energy (i.e., current) returned via a return electrode, which can indicate the progress of the sealing procedure. For example, a rate of change of impedance or phase angle of the return energy may be correlated with known rates of change for different tissue types and thicknesses, thereby enabling a determination of when the sealing is completed. In some exemplary embodiments, a time derivative of current flowing through the tissue may be monitored to determine when to trigger a switch from the sealing procedure to the cutting procedure. Sensors for detecting these values may be provided within ESU 310 or any other component of a surgical system, such as within the surgical instrument itself. In an exemplary embodiment, impedance and/or phase angle are calculated by main controller 313 from current and voltage sensors within sealing circuit 314 and cutting circuit 315. In some embodiments, the system may provide real-time progress data and feedback to a user, which can enable manually triggering the cutting procedure upon being provided an indicator that the sealing procedure is complete. Various combinations of manual-based control and automated/sensor-based controls are envisioned and considered to be within the scope of the present disclosure. Additional details regarding exemplary, but non-limiting, embodiments of electrosurgical signal generators configured for generating and delivering signals to end-effectors for cutting and sealing are disclosed in International Application No. PCT/US2018/066575, and being titled "SIMULTANEOUS ELECTROSURGICAL SEALING AND CUTTING," and published June 27, 2019, which is hereby incorporated by reference herein in its entirety.

FIG. 4 is a schematic perspective view of an embodiment of a pair of opposing jaws of an end effector 430 that can be used for the electrosurgical instrument end effector of FIG. 1. The electrodes of the end effector 430 can be ultimately electrically coupled, through the various electrical conduits, connectors, and interfaces described above, to an ESU 410. In an exemplary embodiment, the ESU 410 can be configured as the ESU 310 of the embodiment of FIG. 3 and be configured to supply voltage and create electrical potential differences to achieve sealing and cutting procedures as further described below. The pair of opposing jaws include a first jaw 431 and a second jaw 432 (with first and second being chosen arbitrarily for ease of reference here and shown in the upper and lower positions in the orientation of the figures). Each of the first and second jaws 431, 432 includes a set of electrodes 433, 434, and 435 on the first jaw 431 and a set of electrodes 436, 437 on the second jaw 432. Thus, in various embodiments the end effector 430 comprises a number of electrodes which can arbitrarily be referred to as "first," "second," "third," "fourth," "fifth," etc. The electrodes on the first jaw respectively generally align with and oppose those on the second jaw; in other words, if the jaws 431, 432 were closed together, electrodes 436 and 433, and electrodes 437 and 434 would respectively align one over the other. Further, an electrically passive (e.g., electrically insulative) surface feature 438 extends longitudinally along a centerline of the second jaw 432 between the electrodes 436, 437, and aligning with electrode 435. For each jaw, two electrodes are disposed toward lateral outer edges of the jaw members (i.e., electrodes 433, 434 on the first jaw 431 and electrodes 436, 437 on the second jaw 432). As further illustrated in FIG. 4, the electrodes may be operably coupled through electrical conduction lines 414-416 to an ESU that can provide signals (as described above) to the electrodes so as to achieve electrosurgical cutting and sealing functionality by selectively establishing cutting and sealing electric potential difference between to return the electrical energy pathway safely away from the device and to an isolated ground at the ESU.

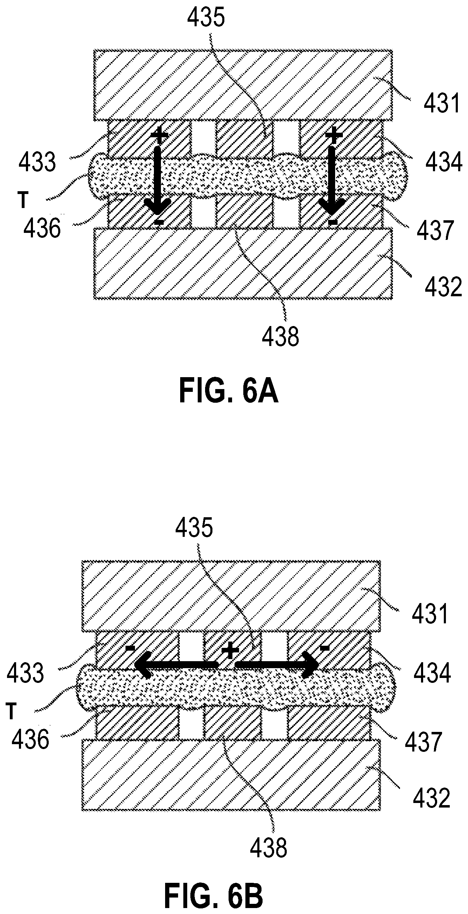

The electrodes 433, 434 of the first jaw 431 are electrically coupled to a sealing circuit 414 configured to provide sealing electrical energy to the electrodes 433, 434 in one operational state, and configured as a cutting return electrode or neutral in other operational states, as further described herein with respect to FIGS. 6A-6D. The electrodes 436, 437 of the second jaw 432 are configured to respectively align with the electrodes 433, 434 in a closed position of the jaws 431, 432 (see FIGS. 6A-6D), and are electrically coupled to a shared return circuit 416 when in a sealing operational state and in one cutting operational state, and are set to a neutral potential in other operational states. The electrode 435 of first jaw 431 is electrically coupled to a cutting circuit 415 configured to provide cutting electrical energy to the electrode 435 in a cutting operational state. An electrically passive (e.g., electrically insulative) surface feature 438 extends longitudinally along a centerline of the second jaw 432 between the electrodes 436, 437 and is in alignment with the electrode 435 in the closed position of the first and second jaws 431, 432.

Thus, in one operating state associated with a sealing procedure, the sealing circuit 414 can supply sealing energy voltage to the electrodes 433, 434 of the first jaw 431 and the shared return circuit 416 can be operated to configure the electrodes 436, 437 to function as sealing return electrodes, respectively to electrodes 433, 434. In an operating state associated with a cutting procedure, the cutting circuit 415 can supply cutting energy voltage to electrode 435 on the first jaw 431, and the electrodes 436, 437 on the second jaw 432 can be used as the cutting return electrodes via the shared return circuit 416. In this way, an electric potential difference is established between the pair of electrodes 433 and 436 and between the pair of electrodes 434 and 437, with the electric potential difference being sufficient to perform a tissue sealing procedure (i.e., sufficient to induce a flow of electrical current through material grasped between the first and second jaws 431, 423 capable of causing a sealing effect in the material), or between the electrode 435 and the pair of electrodes 436, 437 (or alternately with the pair of electrodes 433, 434 as described below), with the electric potential difference being sufficient to perform a tissue cutting procedure (i.e., sufficient to induce a flow of electrical current through material grasped between the first and second jaws 431, 423 capable of causing a cutting effect in the material). Thus, in the operational states described above, the electrodes 436, 437 serve as return electrodes for both the sealing operational state and the cutting operational state. Further in another cutting operational state, the electrodes 433 and/or 434 on the first jaw 431 can be used as return electrodes for a cutting procedure. For example, an electric potential difference is established between an active cut electrode 435 and one or both of electrodes 433/434, with the electrical potential difference being sufficient to perform a cutting procedure. The electric potential differences for cutting operations performed by different sets of electrodes (e.g., electrode 435 to electrodes 436/437 or electrode 435 to electrodes 433/434) can be different due to a difference in distance traversed by electrical energy. Further, alternating between two or more different sets of electrodes for cutting operations can reduce an overall energy consumed for the cutting procedure while ensuring clean cuts without uncut tissue fragments.

As noted above, in some embodiments the electrical signals supplied to the electrodes 433-437 are A/C signals. Thus, in A/C embodiments the voltage (potential difference) between a pair of the electrodes 433-437 that are electrically coupled in a circuit during an operation will alternate over time. Thus, references herein to setting a voltage or potential difference at or between a pair of electrodes 433-437 should be understood, when in the context of A/C signals, as referring to establishing an alternating voltage (potential difference) between the electrodes, and references to the level or magnitude of the voltage (potential difference) should be understood as referring to the A/C voltage (e.g., root-mean-squared voltage (Vrms)) unless otherwise specified or implied by the context. Moreover, references herein or in the figures to circuit elements being at positive or negative potentials should be understood, when in the context of A/C signals, as referring to the polarities of the elements at particular point in time, but those having ordinary skill in the art would understand that the polarities will alternate over time. Furthermore, in an A/C embodiment, as the A/C voltage between a pair of electrodes 433-437 alternates, the direction of current flow between the pair of electrodes 433-437 will also alternate. Thus, references herein to directions of current flow (or directions of electrical energy transmission) should be understood, in an A/C context, as referring to the current at a particular point in time, but those having ordinary skill in the art would understand that the direction of current flow alternates over time.

FIG. 5 is a distal end view of the pair of jaws 431, 432 of the end effector 430 of FIG. 4 shown in a closed position (i.e., the first and second jaws 431, 432 rotated toward one another relative to the axis 424) with biological tissue T grasped between them in accordance with some embodiments. Those having ordinary skill in the art would understand that one or both the first and second jaws 431, 432 is able to rotatably pivot about the pivot axis 424 between the open position in which the first and second jaws 431, 432 are spaced apart from each other and the closed position for grasping biological tissue T between them.

In general, the voltage and current density applied to a biological tissue determines whether cutting or sealing of the tissue is to occur, as a higher voltage and current density is required to achieve the plasma discharge required for cutting of the tissue. A lower current density typically results in less rapid tissue heating, which may result in sealing, which, as used herein, generally occurs due to tissue dehydration, vessel wall shrinkage, and coagulation of blood constituents and collagen denaturation and bonding. A higher current density typically results in the creation of a plasma discharge, which may result in cutting, which as used herein, refers to dissecting of tissue through vaporization, for example. Although electrosurgical sealing signals and electrosurgical cutting signals from the ESU through the relevant circuitry may deliver the same power, they may use different voltage and current levels to do so.

As mentioned above, establishing an electric potential difference between the electrodes on the same jaw (e.g. so as to perform a cutting operation) requires less energy than establishing an electric potential difference the electrodes on opposite jaws and with tissue located between the electrodes. As described above, alternating which pairs or sets of electrodes electric potential differences will be established between during an operating state associated with a cutting procedure, can facilitate the ability to achieve clean, complete, and accurate cuts of tissue without resulting in tissue fragments or uncut pieces.

With reference now to FIGS. 6A-6D, various operational states for the electrodes of the jaw of FIGS. 4 and 5, and electrical pathways between them, is illustrated so as to operate the end effector to perform sealing and cutting procedures in accordance with embodiments of the present disclosure. In FIGs. 6A-6D, pairs of electrodes that are coupled together in a circuit during an operation are indicated by plus "+" and minus "-" signs connected by arrows. The plus and minus signs indicate a relative polarity of the electrodes at a moment in time, with the plus sign indicating the electrode that has the relatively higher potential and the minus sign indicating the electrode having the relatively lower potential. The arrow illustrates a direction of current flow between the pair of electrodes at the moment in time. However, it should be understood that the illustrated polarities of the electrodes are relative, and do not indicate absolute electrical potentials of the electrodes (i.e., an electrode illustrated with a minus sign is not necessarily at a negative absolute potential, but rather the minus sign indicates that its potential is lower than that of the other electrode). Moreover, it should be understood that in contexts in which A/C electrical energy is being supplied, the polarities of the electrodes and the direction of current flow as illustrated in the figures are for a particular moment in time and that the polarities (and hence the direction of current flow) will be reversed at other points in time. It must be noted that the references to "first", "second", and so on are not intended to be limited to the specific components or steps to which they refer, but are merely used for convenience of description. The references to "first", "second", etc. of any component described herein may be changed or modified by those having ordinary skill in the art in light of the embodiments disclosed herein. Further, references to "first operational state", "second cutting state", etc. are not intended to necessarily imply an order of the operational states, which will be understood as being adjustable as desired by an operator of the ESU (and systems associated wherewith).

With reference to FIG. 6A, electrodes 433 and 434 are configured (by, for example, an ESU as described in FIG. 4) to operate in a first sealing operational state. In the first sealing operation state, first sealing electrical energy is supplied between a first pair of electrodes 433 and 436 and between a second pair of electrodes 434 and 437, including establishing an electric potential difference between the first pair of electrodes 433 and 436 and between the second pair of electrodes 434 and 437, with the electrode 435 set at a neutral potential. The potential difference results in flows of electricity from the electrodes 433 and 434 to the electrodes 436 and 437 (at the illustrated point in time), as indicated by the arrows in FIG. 6A. The supplied first sealing electrical energy is configured, via the magnitude of the applied electric potential difference together with other parameters (such as frequency, duty cycle), to induce flows of current through tissue T grasped in between electrodes 433-436 and 434-437 capable of sealing portions of the tissue T. In particular, the magnitude of the electric potential difference may comprise a first sealing voltage, and is configured to seal portions of tissue T grasped in between electrodes 433-436 and 434-437. When the sealing is complete (as determined by, for instance, an impedance sensed in tissue T, or an expiration of a timer, or by any other means), the ESU can initiate one or more cutting operational states.

For the purposes of describing a first cutting state as illustrated in FIG. 6B, in a first cutting operational state first cutting electrical energy is supplied between the electrode 435 and the electrodes 433 and 434, including establishing an electric potential difference between the electrode 435 and at least the electrodes 433 and 434, with the electrodes 436 and 437 set at a neutral potential. The potential difference results in flows of electricity from the electrode 435 to the electrodes 433 and 434 (at the illustrated point in time), as indicated by the arrows in FIG. 6B. The supplied first cutting electrical energy is configured, via the magnitude of the applied electric potential difference together with other parameters (such as frequency, duty cycle), to induce flows of current through the tissue T capable of cutting portions of the tissue T. In particular, the magnitude of the applied electric potential difference may comprise a first cutting voltage, and is configured to cut a portion of tissue T grasped in between electrode 435 and each of electrodes 433 and 434.

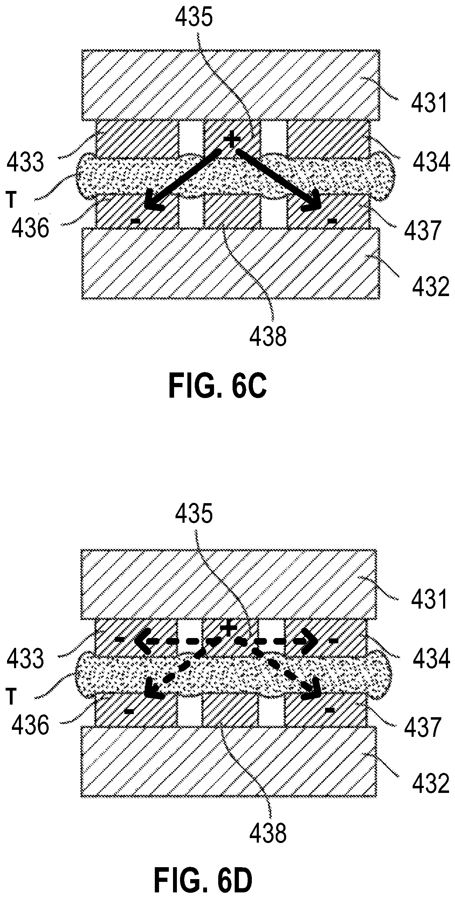

For the purposes of describing a second cutting state as illustrated in FIG. 6C, in the second cutting operational state second cutting electrical energy is supplied between the electrode 435 and the electrodes 436 and 437, including establishing an electric potential difference between the electrode 435 and at least the electrodes 436 and 437, with the electrodes 433, 434 set at the neutral potential. The potential difference results in flows of electricity from the electrode 435 to the electrodes 436 and 437 (at the illustrated point in time), as indicated by the arrows in FIG. 6C. The supplied second cutting electrical energy is configured, via the magnitude of the applied electric potential difference together with other parameters (such as frequency, duty cycle), to induce flows of current through the tissue T capable of cutting portions of the tissue T. In particular, the magnitude of the applied electric potential difference may comprise a second cutting voltage, and is configured to cut a portion of tissue T grasped in between electrodes 435 and electrodes 436 and 437. Adding this conductive pathway (in addition to the configuration described in FIG. 6B) enables tissue T to be cut along multiple pathways, thus ensuring a clean and complete cut.

FIG. 6D illustrates an embodiment wherein the first and second cutting operational states (of FIGS. 6B and 6C respectively) are alternated, such that different portions of tissue T are cut with each cutting operation, thereby enabling a more robust cut. Further, the first and second cutting voltages are different, with the first cutting voltage being less than the second cutting voltage due to the shorter distance being traversed by the electrical energy. Thus, alternating between the two cutting operations can reduce a total amount of energy used to cut the tissue, while ensuring a clean and complete cut without tissue fragments. An alternating pattern of the cutting electrical energy pathway can result in slightly different portions of tissue T being cut with each cutting operation, and a total amount of energy used to cut the tissue being reduced while ensuring a clean and complete cut without tissue fragments. As described herein, an impedance between different sets of electrodes grasping tissue T may be measured, and switching of different operational states may be based on the measured impedance to ensure a complete cut since each operational state cuts the tissue T via different paths. Further, since the second operational state uses less energy (in that the first cut voltage is smaller than the second cut voltage), cycling between the operational states results in less arcing across electrodes. In various exemplary embodiments, the second cutting voltage may range from 300V to 600V, and the first cutting voltage may range from 100V to 400V.

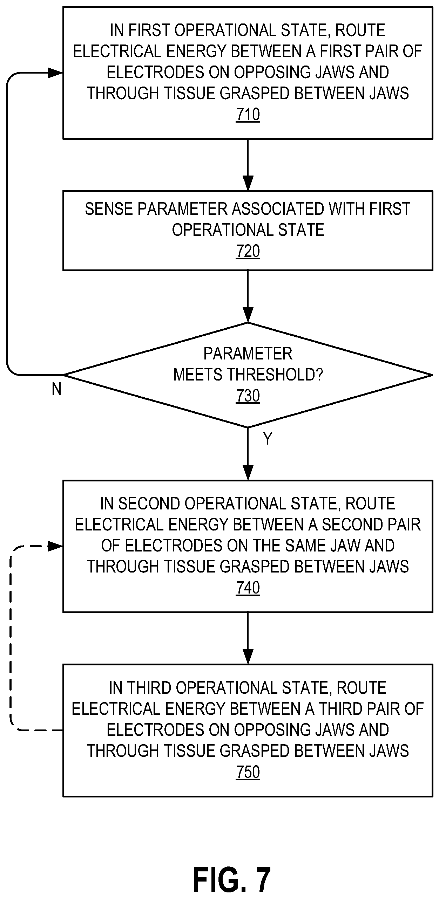

FIG. 7 is a method for controlling electrosurgical instruments in accordance with some embodiments. The methods may be implemented by an electrosurgical system, ESU, electrosurgical instrument, or any other component of an electrosurgical system as described herein in any combination. Although FIG. 7 depicts steps that can be performed in a particular order, in some embodiments, and for purposes of illustration and discussion, the operations discussed herein are not limited to any particular order or arrangement. One skilled in the art, using the disclosures provided herein, will appreciate that various steps of the methods can be omitted, rearranged, combined, and/or adapted in various ways.

With respect to steps 710-750, an electrosurgical instrument comprising a pair of opposing jaws may be selectively operated between first, second, and third operational states. In the first operational state at 710, electrical energy is routed between a first pair of electrodes and through tissue grasped between the jaws, each electrode of the first pair located on differing jaws of the pair of opposing jaws. In this first operational state, the electrical energy is sufficient to seal the tissue grasped between the jaws. At 720, a parameter associated with the first operational state is sensed. The parameter can include, for instance, an impedance measured between the first pair of electrodes (and tissue grasped therebetween). In other embodiments, the parameter can include any parameter that provides an indication of a complete sealing procedure. Thus, at 730, the parameter is compared with a threshold and, if the threshold is not reached, the method continues to perform sealing operation at 710.

However, if and when the parameter meets the threshold, the method continues to In the second operational state at 720, wherein electrical energy is routed between a second pair of electrodes and through the tissue grasped between the jaws, each electrode of the second pair located on a same jaw of the pair of opposing jaws. in this second operational state, the electrical energy is sufficient to cut the tissue grasped between the jaws. An active sealing electrode in the first operational state is set to a return electrode for the second (cutting) operational state. Further, at 750, the electrosurgical instrument may be operated in in a third operational state that comprises routing electrical energy between a third pair of electrodes and through tissue grasped between the jaws, each electrode of the third pair located on differing jaws of the pair of opposing jaws. In the third operational state, the electrical energy is sufficient to cut the tissue grasped between the jaws, and the first pair of electrodes and the third pair of electrodes share a common return electrode. Further, the second and third operational states (at 740 and 750 respectively) can be alternated based on an impedance sensed between one or both of the second pair of electrodes and the third pair of electrodes.

Further modifications and alternative embodiments will be apparent to those of ordinary skill in the art in view of the disclosure herein. Other configurations of electrode placement and polarity can be used and modified to achieve various desired effects. For instance, wiring and positioning of the electrodes can be adjusted to target different energy flows for different types of end effectors.

The embodiments can be implemented in computing hardware (computing apparatus) and/or software, such as (in a non-limiting example) any computer that can store, retrieve, process and/or output data and/or communicate with other computers. The results produced can be displayed on a display of the computing hardware. One or more programs/software comprising algorithms to effect the various responses and signal processing in accordance with various exemplary embodiments of the present disclosure can be implemented by a processor of or in conjunction with the ESU 310 and/or components coupled thereto, and may be recorded on computer-readable media including computer-readable recording and/or storage media. Examples of the computer-readable recording media include a magnetic recording apparatus, an optical disk, a magneto-optical disk, and/or a semiconductor memory (for example, RAM, ROM, etc.). Examples of the magnetic recording apparatus include a hard disk device (HDD), a flexible disk (FD), and a magnetic tape (MT). Examples of the optical disk include a DVD (Digital Versatile Disc), a DVD-RAM, a CD-ROM (Compact Disc - Read Only Memory), and a CD-R (Recordable)/RW.

Further, the systems and the methods may include additional components or steps that were omitted from the diagrams and description for clarity of operation. Accordingly, this description is to be construed as illustrative only and is for the purpose of teaching those skilled in the art the general manner of carrying out the present teachings. It is to be understood that the various embodiments shown and described herein are to be taken as exemplary. Elements and materials, and arrangements of those elements and materials, may be substituted for those illustrated and described herein, parts and processes may be reversed, and certain features of the present teachings may be utilized independently, all as would be apparent to one skilled in the art after having the benefit of the description herein. Changes may be made in the elements described herein without departing from the spirit and scope of the present teachings and following claims. The nature of information depicted in the figures and described herein is exemplary. Those persons having skilled in the art would appreciate modifications to the displays can be made, such as, for example, depending on the number and type of controls desired, the number and/or type of instruments to be used, and/or the functions of the instruments used and the type of fluxes supplied by flux supply units. The various instrument setups depicted in the drawings and described herein are exemplary in nature and the present disclosure contemplates other instrument setups.

This description's terminology is not intended to limit the invention. For example, spatially relative terms-such as "beneath", "below", "lower", "above", "upper", "proximal", "distal", and the like-may be used to describe one element's or feature's relationship to another element or feature as illustrated in the figures. These spatially relative terms are intended to encompass different positions (i.e., locations) and orientations (i.e., rotational placements) of a device in use or operation in addition to the position and orientation shown in the figures. For example, if a device in the figures is turned over, elements described as "below" or "beneath" other elements or features would then be "above" or "over" the other elements or features. Thus, the exemplary term "below" can encompass both positions and orientations of above and below. A device may be otherwise oriented (rotated 90 degrees or at other orientations) and the spatially relative descriptors used herein interpreted accordingly.

It is to be understood that the particular examples and embodiments set forth herein are nonlimiting, and modifications to structure, dimensions, materials, and methodologies may be made without departing from the scope of the present teachings. Other embodiments in accordance with the present disclosure will be apparent to those skilled in the art from consideration of the specification and practice of the invention disclosed herein. It is intended that the specification and examples be considered as exemplary only, with a scope of the following claims being entitled to their broadest interpretation, including equivalents.
The present application also includes the following numbered clauses:
1. An electrosurgical instrument, comprising:
   an electrosurgical energy supply unit; and
   an electrosurgical instrument electrically coupled to the electrosurgical energy supply unit,
   wherein the electrosurgical instrument comprises:
      a first jaw member comprising a first electrode and a second electrode, and
      a second jaw member comprising a third electrode;
   wherein the electrosurgical energy supply unit is configured to control operation of the first, second, and third electrodes such that:
      in a first operational state, a first electric potential difference is established between the first electrode and the third electrode, the second electrode is at a neutral potential, and electrical energy is supplied to the first electrode and returned from the third electrode, and
      in a second operational state, a second electric potential difference is established between the second electrode and the first electrode, the third electrode is at the neutral potential, and electrical energy is supplied to the second electrode and returned from the first electrode.
2. The electrosurgical instrument of clause 1, wherein in a third operational state, a third electric potential difference is established between the second electrode and the third electrode, the first electrode is at the neutral potential, and energy is transmitted between the second electrode and the third electrode.
3. The electrosurgical instrument of clause 2, wherein the electrosurgical energy supply unit is configured to control operation of the first, second, and third electrodes so as to alternate between the second and third operational states.
4. The electrosurgical instrument of clause 3, further comprising a sensor configured to sense an impedance between the second electrode and one or both of the first electrode or the third electrode, wherein the electrosurgical energy supply unit is configured to alternate between the second and third operational states at a frequency based on the impedance.
5. The electrosurgical instrument of clause 2, wherein the first jaw member further comprises a fourth electrode and the second jaw member further comprises a fifth electrode.
6. The electrosurgical instrument of clause 5, wherein in the first operational state, the first electric potential difference is further established between the fourth electrode and the fifth electrode, the second electrode is at the neutral potential, and energy is transmitted between the fourth electrode and the fifth electrode.
7. The electrosurgical instrument of clause 5, wherein in the second operational state, the second electric potential difference is further established between the second electrode and the fourth electrode, the fifth electrode is at the neutral potential, and energy is transmitted between the second electrode and the fourth electrode.
8. The electrosurgical instrument of clause 5, wherein in the third operational state, the third electric potential difference is further established between the second electrode and the fifth electrode, the fourth electrode is at the neutral potential, and energy is transmitted between the second electrode and the fifth electrode.
9. The electrosurgical instrument of clause 2, wherein the second and third electric potential differences are sufficient to induce a cutting effect in tissue grasped between the first and second jaw members.
10. The electrosurgical instrument of clause 9, wherein the second electric potential difference ranges from 300V to 600V, and the third electric potential difference ranges from 100V to 400V.
11. The electrosurgical instrument of clause 1, wherein the first electric potential difference is sufficient to induce a sealing effect in tissue grasped between the first and second jaw members.
12. A method of controlling an electrosurgical instrument, the method comprising:
   selectively operating the electrosurgical instrument between first and second operational states, the electrosurgical instrument comprising a pair of opposing jaws, wherein:
   operating the electrosurgical instrument in the first operational state comprises routing electrical energy between a first pair of electrodes and through tissue grasped between the jaws, each electrode of the first pair located on differing jaws of the pair of opposing jaws; and
   operating the electrosurgical instrument in the second operational state comprises routing electrical energy between a second pair of electrodes and through the tissue grasped between the jaws, each electrode of the second pair located on a same jaw of the pair of opposing jaws.
13. The method of clause 12, wherein in the first operational state, the electrical energy is sufficient to seal the tissue grasped between the jaws.
14. The method of clause 12, wherein in the second operational state, the electrical energy is sufficient to cut the tissue grasped between the jaws.
15. The method of clause 12, wherein an active electrode in the first operational state is set to a return electrode in the second operational state.
16. The method of clause 12, further comprising operating the electrosurgical instrument in a third operational state that comprises routing electrical energy between a third pair of electrodes and through tissue grasped between the jaws, each electrode of the third pair located on differing jaws of the pair of opposing jaws.
17. The method of clause 16, wherein in the third operational state, the electrical energy is sufficient to cut the tissue grasped between the jaws.
18. The method of clause 16, wherein the second pair of electrodes and the third pair of electrodes share a common return electrode.
19. The method of clause 16, further comprising alternating between the second and third operational states.
20. The method of clause 19, wherein the alternating is based on an impedance sensed between one or both of the second pair of electrodes and the third pair of electrodes.
21. A method of controlling an electrosurgical instrument, the method comprising:
   imparting a first cutting signal between a first set of electrodes of an end effector of an electrosurgical instrument, wherein the first set of electrodes are provided on a single jaw member of the end effector;
   imparting a second cutting signal between a second set of electrodes of the end effector, wherein the second set of electrodes are provided on at least two different jaw members of the end effector, and wherein the first and second set of electrodes have at least one electrode in common; and
   alternating between imparting the first and second cutting signals between the first and second sets of electrodes respectively.

## Claims

1. An electrosurgical system, comprising:
an electrosurgical energy supply unit (110, 310, 410); and
an electrosurgical instrument (100, 202) electrically coupled to the electrosurgical energy supply unit,
wherein the electrosurgical instrument comprises:
a first jaw member (431) comprising a first electrode (433, 434) and a second electrode (435), and
a second jaw member (432) comprising a third electrode (436, 437);
wherein the electrosurgical energy supply unit is configured to control operation of the first, second, and third electrodes such that:
in a first operational state, a first electric potential difference is established between the first electrode (433, 434) and the third electrode (436, 437), the second electrode (435) is at a neutral potential, and electrical energy is supplied to the first electrode (433, 434) and returned from the third electrode (436, 437), and
in a second operational state, a second electric potential difference is established between the second electrode (435) and the first electrode (433, 434), the third electrode (436, 437) is at the neutral potential, and electrical energy is supplied to the second electrode (435) and returned from the first electrode (433, 434),
wherein the second electric potential difference is sufficient to induce a cutting effect in material grasped between the first and second jaw members.

2. The electrosurgical system of claim 1, wherein in a third operational state, a third electric potential difference is established between the second electrode (435) and the third electrode (436, 437), the first electrode (433, 434) is at the neutral potential, and energy is transmitted between the second electrode (435) and the third electrode (436, 437).

3. The electrosurgical system of claim 2, wherein the electrosurgical energy supply unit is configured to control operation of the first, second, and third electrodes so as to alternate between the second and third operational states.

4. The electrosurgical system of claim 3, further comprising a sensor configured to sense an impedance between the second electrode (435) and one or both of the first electrode (433, 434) or the third electrode (436, 437), wherein the electrosurgical energy supply unit is configured to alternate between the second and third operational states at a frequency based on the impedance.

5. The electrosurgical system of any of claim 2-4, wherein the first jaw member further comprises a fourth electrode (433, 434) and the second jaw member (432) further comprises a fifth electrode (436, 437).

6. The electrosurgical system of claim 5, wherein in the first operational state, the first electric potential difference is further established between the fourth electrode (433, 434) and the fifth electrode (436, 437), the second electrode (435) is at the neutral potential, and energy is transmitted between the fourth electrode and the fifth electrode.

7. The electrosurgical system of any of claims 5 and 6, wherein in the second operational state, the second electric potential difference is further established between the second electrode (435) and the fourth electrode (433, 434), the fifth electrode (436, 437) is at the neutral potential, and energy is transmitted between the second electrode (435) and the fourth electrode (433, 434).

8. The electrosurgical system of any of claims 5-7, wherein in the third operational state, the third electric potential difference is further established between the second electrode (435) and the fifth electrode (436, 437), the fourth electrode (433, 434) is at the neutral potential, and energy is transmitted between the second electrode (435) and the fifth electrode (436, 437).

9. The electrosurgical system of any of claims 2-8, wherein the third electric potential difference is sufficient to induce a cutting effect in material grasped between the first and second jaw members.

10. The electrosurgical system of any of claims 2-9, wherein the third electric potential difference ranges from 300V to 600V, and the second electric potential difference ranges from 100V to 400V.

11. The electrosurgical system of any of claims 1-10, wherein the first electric potential difference is sufficient to induce a sealing effect in material grasped between the first and second jaw members.

12. An electrosurgical energy supply unit, comprising:
a power source;
sealing circuitry configured to generate a sealing electrical signal;
cutting circuitry configured to generate a first cutting electrical signal and a second cutting electrical signal;
relay circuity configured to selectively apply the sealing electrical signal, the first cutting signal, and the second cutting signal between electrodes of an electrosurgical instrument electrically coupled to the electrosurgical energy supply unit, the electrodes comprising a first electrode (433, 434) coupled to a first jaw member (431) of the electrosurgical instrument, a second electrode (435) coupled to the first jaw member (431), and a third electrode (436, 437) coupled to a second jaw member (432) of the electrosurgical instrument; and
a controller configured to cause the relay circuitry to:
in a first operational state, apply the sealing electrical signal between the first electrode (433, 434) and the third electrode (436, 437); and
in a second operation state, apply the first cutting electrical signal between the first electrode (433, 434) and the second electrode (435), and
in a third operational state, apply the second cutting electrical signal between the second electrode (435) and the third electrode (436, 437).

13. The electrosurgical energy supply unit of claim 12, wherein the electrosurgical energy supply unit is configured to, in response to an input to perform a cutting operation, alternate between the second and third operational states.

14. The electrosurgical energy supply unit of claim 13, wherein the controller is configured to receive impedance information indicating one or both of sensed impedance between the second electrode (435) and the first electrode (433, 434) or sensed impedance between the second (435) electrode and the third electrode (436, 437), and to, during the cutting operation, alternate between the second and third operational states based on the impedance information.

15. The electrosurgical energy supply unit of any of claims 12 through 14,
wherein the relay circuity comprises:
a first conductor (415) configured to be electrically connected to the second electrode (435) in a coupled state of the electrosurgical instrument to the electrosurgical energy supply unit;
a second conductor (414) configured to be electrically connected to the first electrode (433, 434) in the coupled state of the electrosurgical instrument; and
a third conductor (416) configured to be electrically connected to the third electrode (436, 437) in the coupled state of the electrosurgical instrument;
wherein in the first operational state, the relay connects the second and third conductors to the sealing circuit to apply the sealing electrical signal through the second and third conductors;
wherein in the second operation state, the relay connects the first and second conductors to the cutting circuit to apply the first cutting electrical signal through the first and second conductors; and
wherein in the third operational state, the relay connects the first and third conductors to the cutting circuit to apply the second cutting electrical signal through the first and third conductors.
